# EUROPEAN PATENT SPECIFICATION

(11) **EP 3 360 866 B1**
(45) Date of publication and mention of the grant of the patent: **02.01.2019**
(21) Application number: 17156130.1
(22) Date of filing: 14.02.2017
(51) Int. Cl.: C07D 277/40, A61P 13/10, A61K 31/426

(54) **MIRABEGRON PRODRUGS**
MIRABEGRON-PRODRUGS
PROMÉDICAMENTS MIRABEGRON

(43) Date of publication of application: 15.08.2018
(73) Proprietor: Alfred E. Tiefenbacher (GmbH & Co. KG), 22767 Hamburg (DE)
(72) Inventor: Staver, Ruslan, 22607 Hamburg (DE); Aicher, Daniel, 10823 Berlin (DE)
(74) Representative: Hamm&Wittkopp Patentanwälte PartmbB

(56) References cited:
- EP-A1- 1 028 111
- EP-A1- 1 440 969
- EP-A1- 1 559 427
- US-A- 5 684 018

## Description

The present invention relates to prodrugs of Mirabegron and their use in therapy.

Mirabegron is a β-3 adrenergic agonist indicated for the treatment of overactive bladder. Extended-release tablets containing 25 mg or 50 mg Mirabegron are commercially available under the tradenames Betmiga® and Myrbetriq®. The use of Mirabegron for the treatment of overactive bladder is described in European patent EP 1 559 427.

Initially, Mirabegron was developed as a remedy for diabetes. European patent EP 1 028 111 discloses the preparation of the dihydrochloride salt of Mirabegron (example 41) and its use for the treatment of diabetes mellitus. According to European patent application EP 1 440 969, the dihydrochloride salt has strong hygroscopicity and is unstable, which poses problems for converting the drug into solid unit dosage forms. As a solution to this problem, the application suggests the crystalline forms α and β of Mirabegron (the free base), which are much less hygroscopic than the dihydrochloride salt.

The free base of Mirabegron can be formulated as a stable matrix-type extended-release tablet containing polyethylene oxide as retarding polymer as well as ferric oxide, as described in European patent EP 1 205 190. However, the commercially available tablets, which are prepared by using the technique described in EP 1 205 190, provide a relatively low bioavailability of the drug, which is even lower if the tablet is taken together with food. Hence, there was a need for improving the oral bioavailability of Mirabegron.

As a solution to the decreased oral bioavailability if Mirabegron is administered together with food, European patent application EP 2 345 410 suggests an extended-release tablet that contains a hydrogel-forming polymer, preferably polyethylene oxide, and an additive that ensures penetration of water into the pharmaceutical composition. Preferably, the additive is selected from polyethylene glycol, polyvinylpyrrolidone, mannitol, sorbitol, sodium chloride, etc. European patent application EP 2 554 168 discloses a multi-layer tablet comprising a Mirabegron-containing layer and release-controlling layers, a tablet in which the drug is contained in a gel formulation composed of gums like locust bean gum and xanthan gum, as well as osmotic pump type tablet formulations. Hence, various tablet formulations have been described in the state of the art in order to minimize the food effect and, thus, enhance the oral bioavailability of Mirabegron.

It was an object of the present invention to provide a pharmaceutical composition containing Mirabegron with improved oral bioavailability. This object is solved by the subject matter as defined in the claims.

It was found that a prodrug of Mirabegron according to the present invention improves the oral bioavailability of the drug. Thus, the present invention relates to a prodrug of Mirabegron represented by the following formula (I) 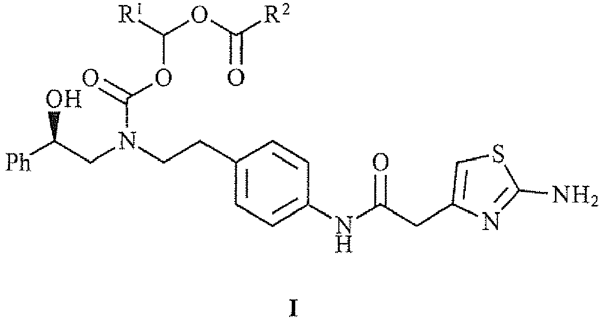 wherein
R¹ represents hydrogen or an optionally substituted, saturated or unsaturated alkyl, and
R² represents an optionally substituted, saturated or unsaturated alkyl, optionally substituted, saturated or unsaturated cycloalkyl, optionally substituted, saturated or unsaturated (cycloalkyl)alkyl, optionally substituted, saturated or unsaturated heterocyclyl, optionally substituted, saturated or unsaturated (heterocyclyl)alkyl, optionally substituted aryl, or optionally substituted heteroaryl,
wherein the substituent of the optionally substituted alkyl, cycloalkyl, (cycloalkyl)alkyl, heterocyclyl, (heterocyclyl)alkyl, aryl and heteroaryl is selected from halogen (F, Cl, Br or I), C₁₋₆-alkoxy, preferably C₁₋₄-alkoxy, more preferably C₁₋₂-alkoxy, phenoxy, (C₁₋₄ alkyl)₂ amino and phenyl,
or a pharmaceutically acceptable salt thereof or a solvate thereof.

In case R¹ is not hydrogen, the prodrugs of formula (I) are obtained as a diastereomeric mixture. The diastereomers can be separated by using HPLC or by fractional crystallization.

In the prodrugs of the present invention, a carbamate is formed with Mirabegron's secondary amine (the N_{phenethyl} atom). A prodrug is an enzymatically reversible derivative of a drug, i.e. a compound that, upon introduction in the appropriate biological system, reverts back to the parent molecule by virtue of enzymatic cleavage. The secondary amine atom of Mirabegron is derivatized as a carbamate, whereby the polar amino group is masked and the drug absorption and, thus, oral bioavailability is enhanced. However, since the carbamate after absorption needs to be hydrolyzed to carbamic acid and the corresponding alcohol, and since the rates of hydrolysis of secondary amines are very slow, an ester moiety is linked to the carbamate moiety in the prodrugs of the present invention in order to initiate the regeneration of the parent amine Mirabegron by an esterase-catalyzed hydrolysis of the ester moiety. The use of a mixed ester-carbamate group as prodrug motif was disclosed in US 5,684,018.

In the prodrugs of the present invention, usually the saturated alkyl group is C₁₋₇ alkyl, preferably C₁₋₅ alkyl, the unsaturated alkyl group is C₂₋₇ alkenyl, preferably C₂₋₅ alkenyl, the saturated or unsaturated cycloalkyl group is cyclo C₃₋₆ alkyl, the saturated or unsaturated (cycloalkyl)alkyl group is (cyclo-C₃₋₆ alkyl)C₁₋₅ alkyl, the saturated or unsaturated heterocyclyl group is cyclo C₃₋₆ heterocyclyl, in which 1 to 3 carbon atoms of the ring are replaced by O, N or S, the saturated or unsaturated (heterocyclyl)alkyl group is (cyclo C₃₋₆ heterocyclyl)C₁₋₅alkyl, in which 1 to 3 carbon atoms of the ring are replaced by O, N or S, the aryl group is C₆ or C₁₀ aryl, and the heteroaryl group is C₆ or C₁₀ heteroaryl, in which 1 to 3 carbon atoms of the ring are replaced by O, N or S.

It is more preferred that R¹ represents hydrogen or an unsubstituted, saturated alkyl, and R² represents an unsubstituted, saturated alkyl, or an unsubstituted aryl. For example, R¹ can be selected from hydrogen, methyl, ethyl, n-propyl, iso-propyl, n-butyl, sec-butyl and tert-butyl, and R² can be selected from methyl, ethyl, propyl (n- or iso-), butyl (n-, sec- or tert-), n-pentyl, 2-methylbutyl, 3-methylbutyl and phenyl. Most preferably, R¹ is hydrogen or methyl, and R² is selected from methyl, ethyl, n-propyl, iso-propyl, n-butyl, sec-butyl, n-pentyl and phenyl. Especially preferred is a prodrug in which R¹ is methyl and R² is iso-propyl (Enacarbil-N_{phenethyl}-Mirabegron). Also especially preferred are prodrugs in which R¹ is hydrogen and R² is n-pentyl (hexanoyloxy(methoxy)carbonyl-N_{phenethyl}-Mirabegron) or phenyl (benzoyloxy(methoxy)carbonyl-N_{phenethyl}-mirabegron),

The prodrugs of the present invention may be prepared as outlined in the following scheme:

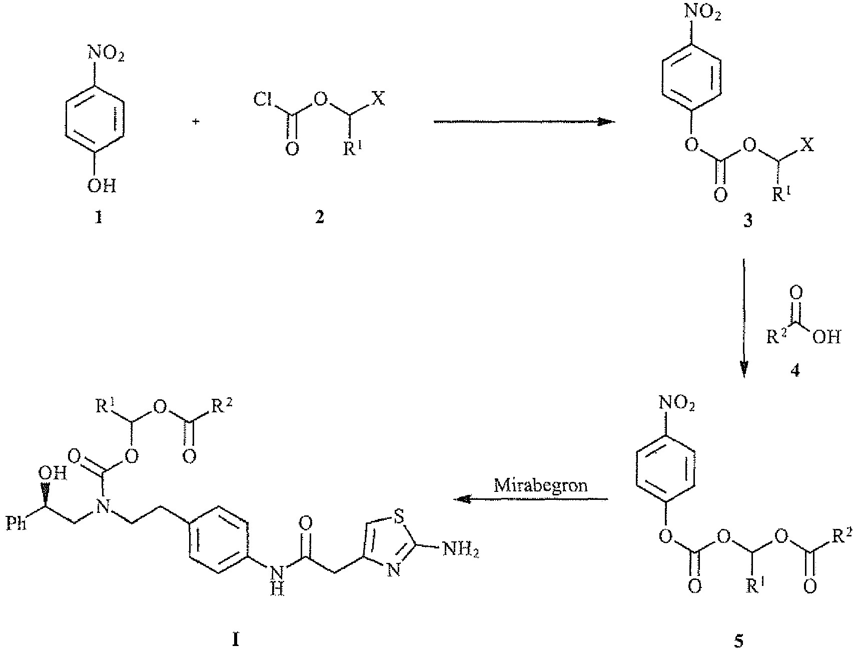

The key intermediate of the process is the mixed carbonate 5 with a para-nitrophenyl moiety that reacts with Mirabegron to yield the N_{phenethyl}-Mirabegron derivatized prodrug I. Hence, the present invention relates to a process for preparing a prodrug of formula (1) comprising the step of reacting Mirabegron and a carbonate compound represented by the following formula (5) 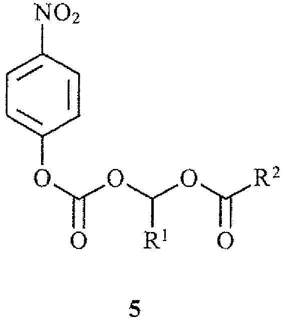 wherein R¹ and R² are as defined above.

As an alternative to the mixed carbonate 5, chloroformates and carbamates with, e.g., a succinimide moiety of the following formulas may be used:

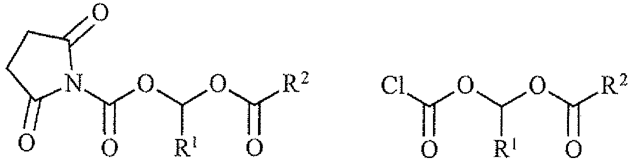

The mixed carbonate compound 5 may be prepared by reacting a carbonate compound and an acid of the following formulas 3 and 4 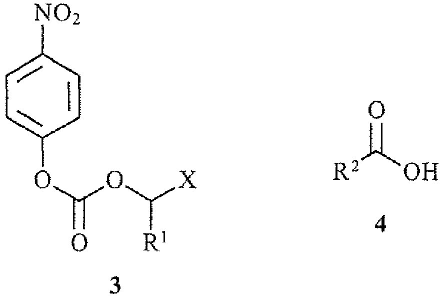 wherein R¹ and R² are as defined above, and X is a leaving group, e.g. Cl, Br, I, alkylsulfonyloxy or arylsulfonyloxy (e.g. methanesulfonyloxy or para-toluene-sulfonyloxy).

The carbonate compound 3 may be prepared by reacting para-nitrophenol and a compound of the following formula 2 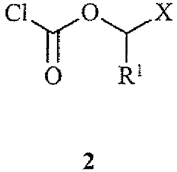 wherein R¹ and X are as defined above.

The present invention further relates to a pharmaceutical composition for oral administration comprising a prodrug of formula (I). Preferably, the pharmaceutical composition for oral administration is an extended-release tablet containing the prodrug evenly dispersed within a retarding polymer. Preferably, the extended-release matrix contains polyethylene oxide and/or hydroxypropylmethyl cellulose as a retarding agent as well as polyethylene glycol. The pharmaceutical composition is suitable for the treatment of overactive bladder. The prodrug of the present invention may be used for the treatment of overactive bladder.

The chemical stability of the prodrugs of formula (I) was tested in simulated gastric fluid (pH 2), fasted-state simulated intestinal fluid (pH 6.5), fed-state simulated intestinal fluid (pH 5.0) and in phosphate buffer (pH 7.5). No hydrolytical cleavage of the prodrug was observed within 48 hours under all conditions.

Compared to the parent compound Mirabegron, it was found that the Tₘₐₓ is earlier and the Cₘₐₓ is higher after the oral administration of Enacarbil-N_{phenethyl}-Mirabegron to Sprague Dawley rats. The half-life of this prodrug in rat blood plasma was less than 0.5 h and in human blood plasma less than 2.5 h. Almost 100 % esterase cleavage was found in rat blood plasma after 1 h, whereas about 70 % of the prodrug was cleaved within 4 hours in human blood plasma.

The half-life of Benzoyloxy(methoxy)carbonyl-N_{phenethyl}-Mirabegron in rat blood plasma and in human blood plasma was less than 0.5 h. Almost 100 % esterase cleavage was found in rat blood plasma after 1 h, whereas almost 100 % of the prodrug was cleaved within 4 hours in human blood plasma.

Hence, it could be demonstrated that the prodrugs of the present invention have excellent chemical stability, i.e. they are not hydrolyzed during the passage through the gastrointestinal tract, that they are absorbed faster than the parent compound Mirabegron and that the parent compound is readily set free by esterases after penetration through the biological barrier.

Other prodrugs are represented by the following formula (II) 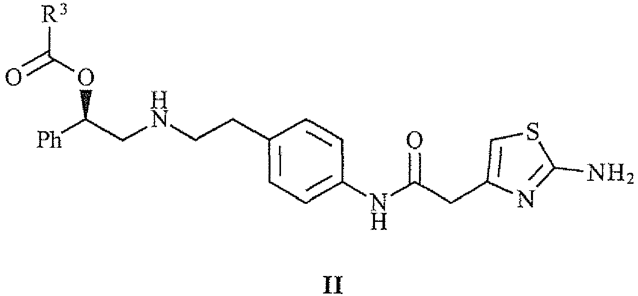 wherein R³ represents an optionally substituted, saturated or unsaturated alkyl, optionally substituted, saturated or unsaturated cycloalkyl, optionally substituted, saturated or unsaturated (cycloalkyl)alkyl, optionally substituted, saturated or unsaturated heterocyclyl, optionally substituted, saturated or unsaturated (heterocyclyl)alkyl, optionally substituted aryl, or optionally substituted heteroaryl,
or a pharmaceutically acceptable salt thereof or a solvate thereof.

In the prodrugs of formula (II), usually the saturated alkyl group is C₁₋₇ alkyl, preferably C₁₋₅ alkyl, the unsaturated alkyl group is C₂₋₇ alkenyl, preferably C₂₋₅ alkenyl, the saturated or unsaturated cycloalkyl group is cyclo-C₃₋₆ alkyl, the saturated or unsaturated (cycloalkyl)alkyl group is (cyclo-C₃₋₆ alkyl)C₁₋₅ alkyl, the saturated or unsaturated heterocyclyl group is cyclo-C₃₋₆ heterocyclyl, in which 1 to 3 carbon atoms of the ring are replaced by O, N or S, the saturated or unsaturated (heterocyclyl)alkyl group is (cyclo-C₃₋₆ heterocyclyl)C₁₋₅ alkyl, in which 1 to 3 carbon atoms of the ring are replaced by O, N or S, the aryl group is C₆ or C₁₀ aryl, and the heteroaryl group is C₆ or C₁₀ heteroaryl, in which 1 to 3 carbon atoms of the ring are replaced by O, N or S. Preferably, R³ represents an unsubstituted, saturated alkyl, an unsubstituted cycloalkyl or an unsubstituted aryl; more preferably, R³ is selected from methyl, ethyl, n-propyl, iso-propyl, n-butyl, sec-butyl, tert-butyl, cyclopropyl, cyclopentyl, cyclohexyl and phenyl.

The prodrug of formula (II) may be prepared by protecting the nitrogen atoms of Mirabegron, e.g. as tris(tert-butyl carbamate), and subsequent reaction of the nitrogen-protected Mirabegron derivative with the corresponding activated acid derivative (e.g., with the respective acyl chloride) followed by deprotection. The invention is further illustrated by reference to the following examples.

### Examples

### Example 1. Enacarbil-N_{phenethyl}-Mirabegron

### Step a: 1-Chloroethyl-p-nitrophenyl carbonate

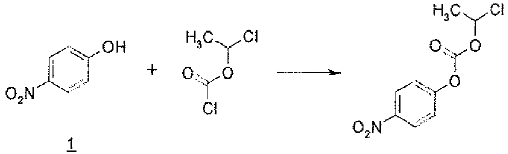

To an ice-cold reaction mixture containing p-nitrophenol (1.39 g, 10 mmol) and triethylamine (10 mmol) in dichloromethane (50 mL) was added 1-chloroethyl chloroformate (1.2 mL, 11 mmol) in 10 ml of dichloromethane. The mixture was stirred at 0°C for 30 min and then at room temperature for 1 hour. The reaction mixture was diluted with water; the organic layer was washed 5-7 times with saturated sodium bicarbonate solution (until the color of the aqueous layer is not very strong yellow-colored) and dried. After removing the solvent under reduced pressure, the residue was dissolved in ethyl acetate, washed 3x with saturated sodium bicarbonate solution, 2x with 10% citric acid, and 2-3x with brine. The organic layer was dried over anhydrous sodium sulfate and evaporated under reduced pressure to give 2,4 g (97%) of the title compound as an off-white solid.
**Yield:**
12 g (M 245.6), 48.9 mmol

### Step b: Isobutoxyethyl-p-nitrophenyl carbonate

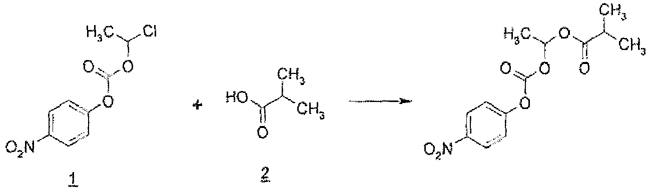

| | | | |
|---|---|---|---|
| 1 M 245.6 | 6.0 g | 25.0 mmol | 1.0 eq |
| 2 M 88.1, d 0.95 | 12 ml | 125.0 mmol | 5.0 eq |
| Ag₂CO₃ M 275.7 | 10g | 37.5 mmol | 1.5 eq |
| dry toluene | 125 ml | | |

To the solution of 1-chloroethyl-p-nitrophenyl carbonate and isobutyric acid in toluene, silver carbonate is added. The mixture is stirred under reflux overnight in the dark (aluminum foil is used to cover the equipment). The reaction mixture was allowed to cool to room temperature and filtered through a pad of Celite. The filtrate was washed 5x with saturated sodium bicarbonate solution. After removing the solvent under reduced pressure, the residue was dissolved in ethyl acetate, washed 3x with saturated sodium bicarbonate solution and 1-2x with brine. The organic layer was dried over anhydrous sodium sulfate and evaporated under reduced pressure. The isolated substance contains only 20% of the title compound and 80% of an unknown impurity, probably an isobutyric ester.
**Yield:**
5 g (purity 20%)

### Step c: Enacarbil-N_{phenethyl}-Mirabegron

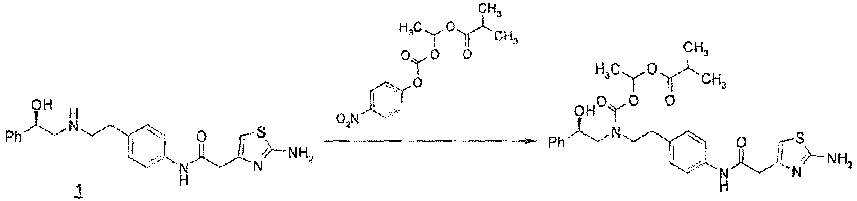

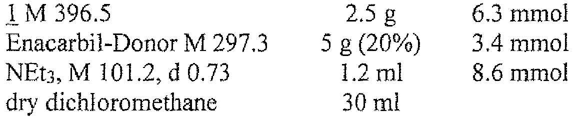

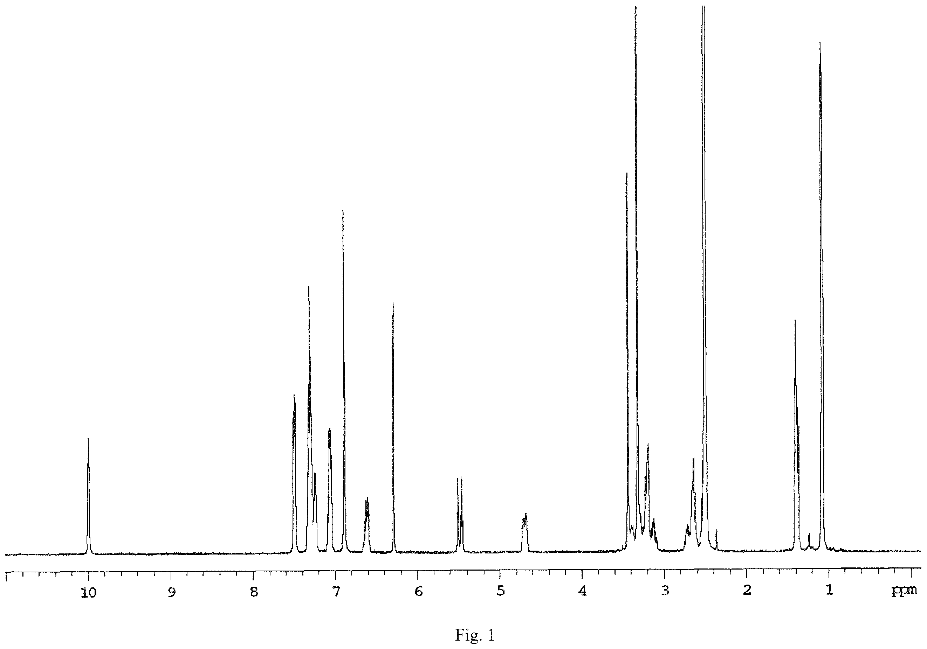

Mirabegron was dissolved in dry dichloromethane. Then triethylamine and the crude Enacarbil-Donor from step b) were added. After stirring for 7 days the reaction mixture was quenched with 100 ml water and a small amount of sodium hydrogen carbonate solution was added. The aqueous layer was extracted twice with dichloromethane. The combined organic layers were evaporated in vacuum. The first purification was achieved by preparative HPLC: Phenomenex Luna Phenyl-Hexyl: MeCN/H₂O/TFA 70:30:0.1. The product fractions were combined and acetonitrile was evaporated in vacuum. The aqueous layer was carefully basified to pH 6 with saturated sodium hydrogen carbonate solution and then brine was added and the aqueous layer was extracted several times with ethylacetate. Further purification was achieved by preparative HPLC: Phenomenex Luna Phenyl-Hexyl: MeCN/H₂O/TFA 50:50:0.1. The product fractions were combined and acetonitrile was evaporated in vacuum. The aqueous layer was carefully basified to pH 6 with saturated sodium hydrogen carbonate solution and then brine was added and the aqueous layer was extracted several times with ethylacetate. The residue was treated with diethyl ether and dried in vacuum and a white solid was obtained.
**Yield:**
0.3 g
Melting point (m.p.): 58-62°C. MS (ESI) m/z 555.2299 (M+H)⁺.
¹H-NMR (DMSO-d6): Figure 1 (1 Diastereomer)

### Example 2. Benzoyloxy(methoxy)carbonyl-Nphenethyl-Mirabegron

### Step a: 1-Chloromethyl-p-nitrophenyl carbonate

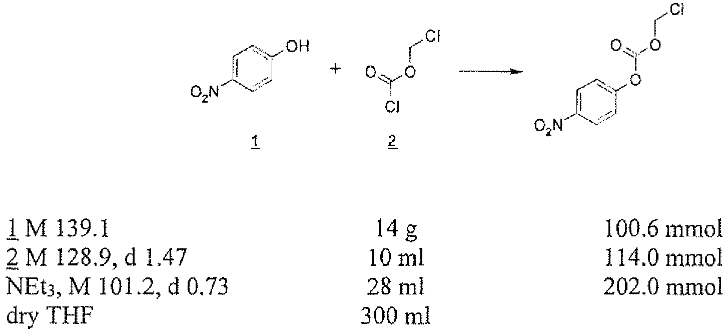

To a -40 °C reaction mixture containing p-nitrophenol and chloromethyl chloroformate in tetrahydrofuran was added triethylamine in one portion. The mixture was stirred at -40°C for 30 min. The solid was filtered off and the filtrate was diluted with ethyl acetate; the organic layer was washed 5 times with a mixture of water, brine and sodium hydrogen carbonate solution. The organic layer was dried over anhydrous sodium sulfate and evaporated under reduced pressure.
**Yield:**
20 g (M 231.6), 86.3 mmol, 86%, yellow oil

### Step b: 1-Iodomethyl-p-nitrophenyl carbonate

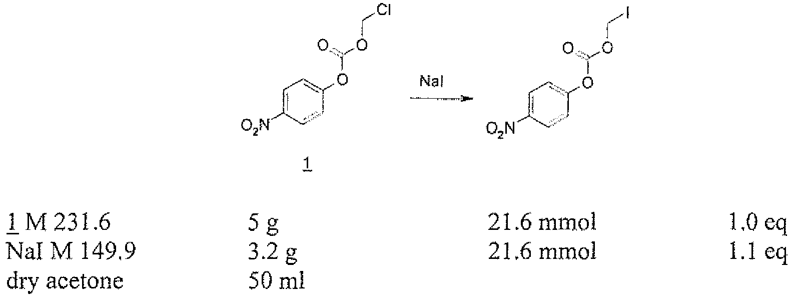

To a solution of 1 in acetone was added NaI in one portion and the mixture was stirred at 50°C overnight. The solvent was evaporated and the residue was dissolved in diethylether. The organic phase was washed 3 times with brine and dried over sodium sulfate.
**Yield:**
6 g (M 323.0), 18.6 mmol, 86%

### Step c: Benzoyloxy(methoxy)carbonyl-4-nitrophenol

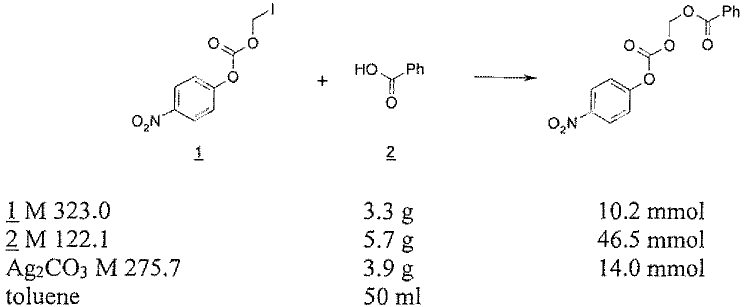

To the solution of 1 and 2 in toluene, silver carbonate is added. The mixture is stirred under reflux 2 h in the dark (aluminum foil is used to cover the equipment). The reaction mixture was allowed to cool to room temperature and filtered. The filtrate was washed with water and brine and dried over anhydrous sodium sulfate. After removing the solvent under reduced pressure, the residue was dissolved in diethylether, washed 3 x with sodium bicarbonate solution and 1 x with brine. The organic layer was dried over anhydrous sodium sulfate and evaporated under reduced pressure.
**Yield:**
2.8 g (M 317.3), 8.8 mmol, 86%, yellow oil

### Step d: Benzoyloxy(methoxy)carbonyl-N_{phenethyl}-Mirabegron

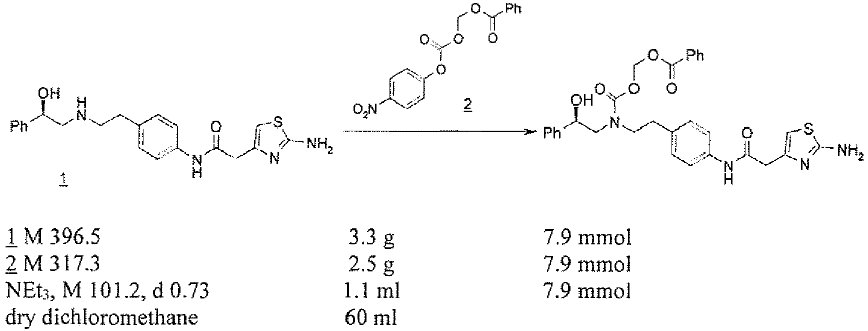

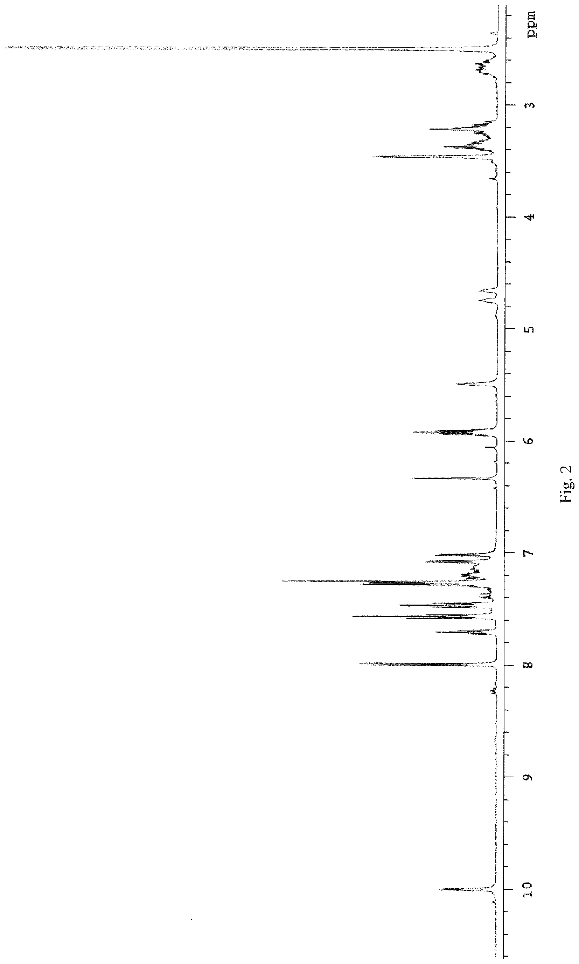

Mirabegron was dissolved in dry dichloromethane. Then triethylamine and benzoyloxy(methoxy)carbonyl-4-nitrophenol were added. After stirring for 2 days the reaction mixture was quenched with 100 ml water and a small amount of sodium hydrogen carbonate solution was added. The aqueous layer was extracted twice with dichloromethane. The combined organic layers were evaporated in vacuum. The first purification was achieved by preparative HPLC: Phenomenex Luna Phenyl-Hexyl: MeCN/H₂O/TFA 60:40:0.1. The product fractions were combined and acetonitrile was evaporated in vacuum. The aqueous layer was carefully basified to pH 6 with saturated sodium hydrogen carbonate solution and then brine was added and the aqueous layer was extracted several times with ethylacetate. Further purification was achieved by repeated preparative HPLC: Phenomenex Luna Phenyl-Hexyl: MeCN/H₂O/TFA 60:40:0.1. The product fractions were combined and acetonitrile was evaporated in vacuum. The aqueous layer was carefully basified to pH 6 with saturated sodium hydrogen carbonate solution and then brine was added and the aqueous layer was extracted several times with ethylacetate. The residue was treated with diethyl ether and dried in vacuum and a light yellow solid was obtained.
**Yield:**
650 mg (M 574.7), 1.1 mmol, 14 %, light yellow solid.
m.p.: 71-74°C. MS (ESI) m/z 575.1961 (M+H)⁺.
¹H-NMR (DMSO-d6): Figure 2

### Example 3. Hexanoyloxy(methoxycarbonyl)-N_{phenethyl}-Mirabegron

Steps a and b correspond to steps a and b of Example 2.

### Step c: Hexanoyloxy(methoxy)carbonyl-4-nitrophenol

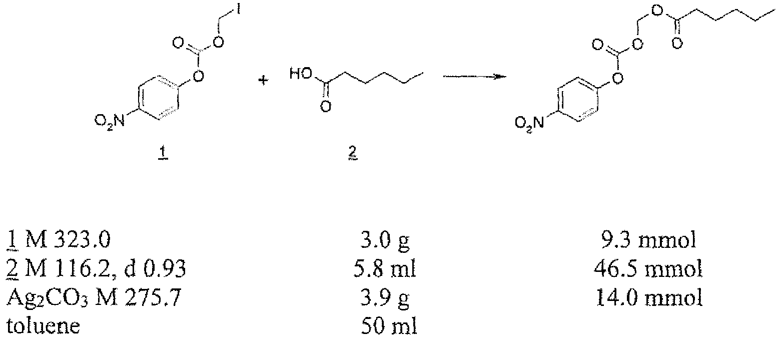

To the solution of 1 and 2 in toluene, silver carbonate is added. The mixture is stirred under reflux 2 h in the dark (aluminum foil is used to cover the equipment). The reaction mixture was allowed to cool to room temperature and filtered. The filtrate was washed with water and brine and dried over anhydrous sodium sulfate. After removing the solvent under reduced pressure, the residue was dissolved in diethylether, washed 3 x with sodium bicarbonate solution and 1 x with brine. The organic layer was dried over anhydrous sodium sulfate and evaporated under reduced pressure.
**Yield:**
3.5 g (M 311.3), 8.4 mmol, 90%, yellow oil

### Step d: Hexanoyloxy(methoxycarbonyl)-N_{phenethyl}-Mirabegron

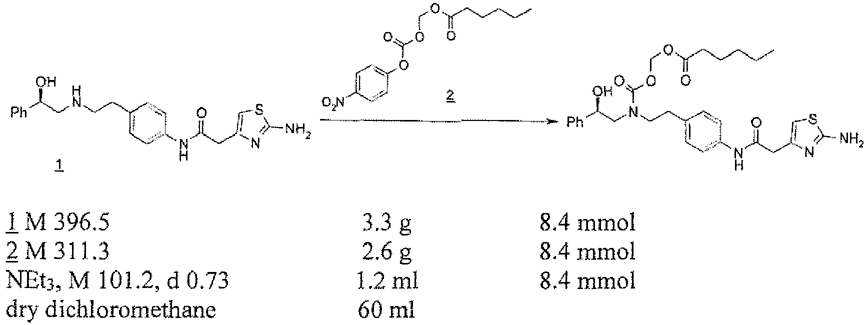

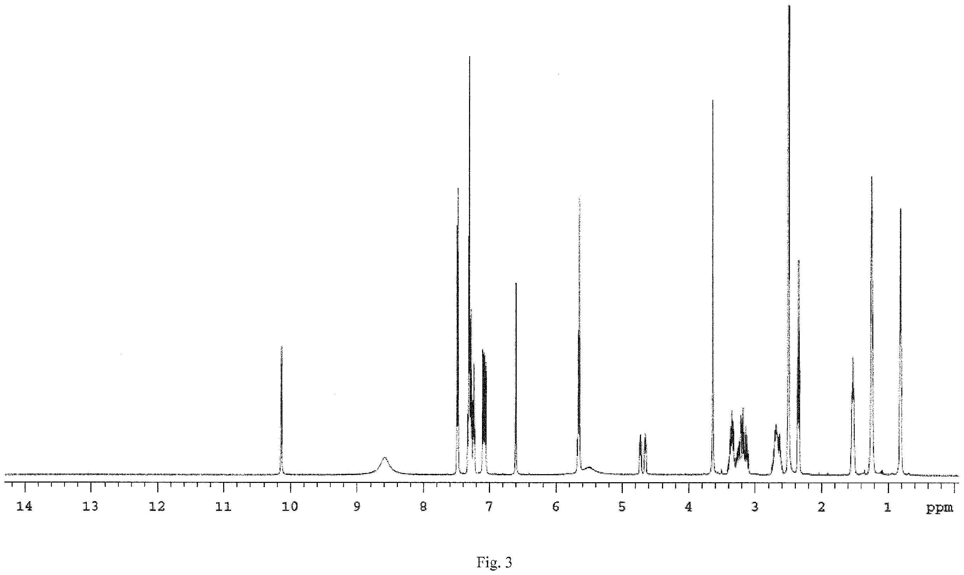

Mirabegron was dissolved in dry dichloromethane. Then triethylamine and hexanoyloxy(methoxy)carbonyl-4-nitrophenol were added. After stirring for 2 days the reaction mixture was quenched with 100 ml water and a small amount of sodium hydrogen carbonate solution was added. The aqueous layer was extracted twice with dichloromethane. The combined organic layers were evaporated in vacuum. The first purification was achieved by preparative HPLC: Phenomenex Luna Phenyl-Hexyl: MeCN/H₂O/TFA 60:40:0.1. The product fractions were combined and acetonitrile was evaporated in vacuum. The aqueous layer was carefully basified to pH 6 with saturated sodium hydrogen carbonate solution and then brine was added and the aqueous layer was extracted several times with ethylacetate. Further purification was achieved by repeated preparative HPLC: Phenomenex Luna Phenyl-Hexyl: MeCN/H₂O/TFA 60:40:0.1. The product fractions were combined and acetonitrile was evaporated in vacuum. The aqueous layer was carefully basified to pH 6 with saturated sodium hydrogen carbonate solution and then brine was added and the aqueous layer was extracted several times with ethylacetate. The residue was treated with diethyl ether and dried in vacuum and a white solid was obtained.
**Yield:**
200 mg (M 568.7), 0.35 mmol, 4%, white solid
m.p.: 58-63°C. MS (ESI) m/z 569.2412 (M+H)⁺.
¹H-NMR (DMSO-d6): Figure 3

### Reference Example 4. O-Pivaloyl-Mirabegron

### Step a: N,N',N'-Mirabegron-tris(t-butylcarbamate)

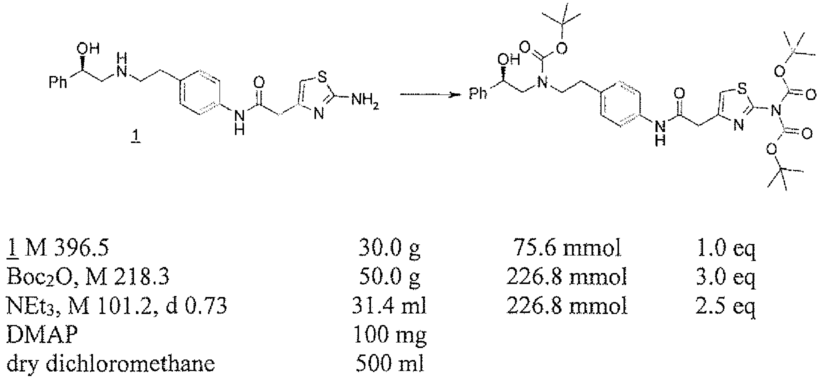

Mirabegron was dissolved in dry dichloromethane. Then triethylamine and Boc₂O were added. DMAP was added and the reaction was stirred overnight. Additional Boc₂O and NEt₃ was added and the mixture was stirred one more night. The reaction mixture was quenched with 500 ml water and brine was added. The aqueous layer was extracted twice with dichloromethane. The combined organic layers were evaporated in vacuum. Purification was achieved by repeated column chromatography: silica gel: chloroform/methanol 97.5:2.5.
**Yield:**
17 g (M 696.9), 24.4 mmol

### Step b: O-Pivaloyl-N,N',N'-Mirabegron-tris(t-butylcarbamate)

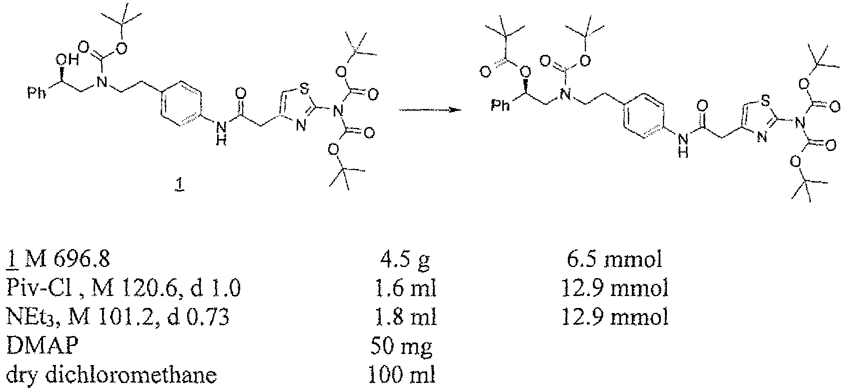

1 was dissolved in dry dichloromethane. Then triethylamine, pivaloyl chloride and DMAP were added and the reaction was stirred overnight. The reaction mixture was quenched with 100 ml water and brine was added. The aqueous layer was extracted twice with dichloromethane. The combined organic layers were evaporated in vacuum. Purification was achieved by preparative HPLC: Phenomenex Luna Phenyl-Hexyl: MeCN/H₂O/TFA 85:15:0.1. The aqueous layer was carefully basified to pH 6 with saturated sodium hydrogen carbonate solution and acetonitrile was removed in vacuum. Then brine was added and the aqueous layer was extracted several times with ethylacetate. The combined organic layers were dried over sodium sulfate and evaporated in vacuum.
**Yield:**
1.6 g (M 781,0), 2.1 mmol

### Step c: O-Pivaloyl-Mirabegron

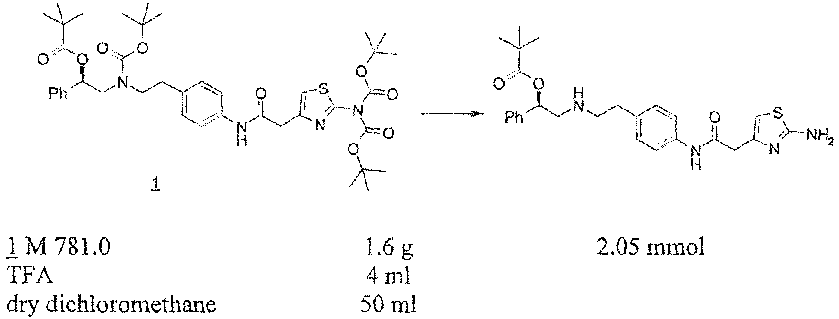

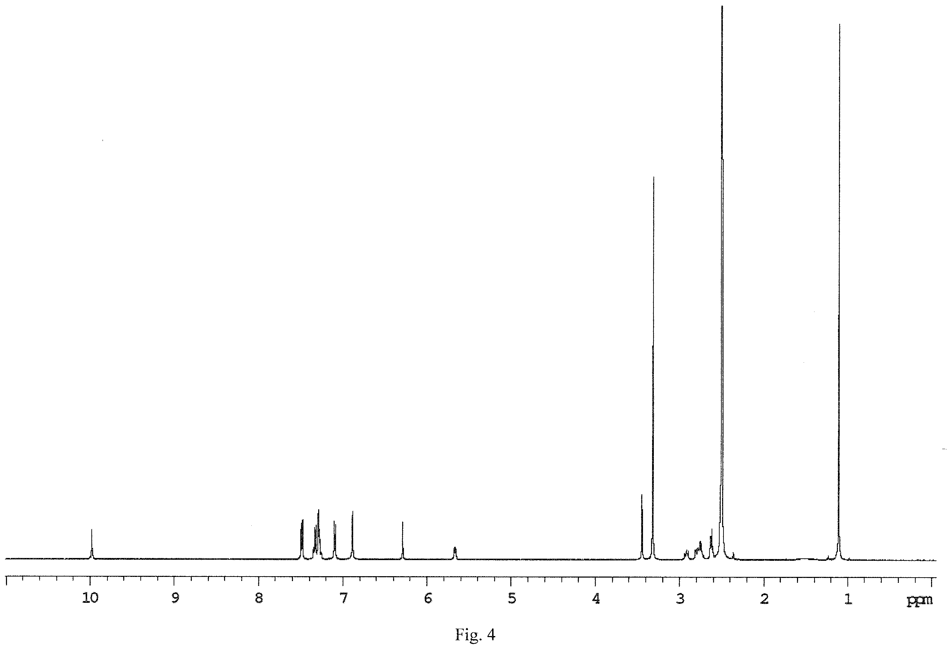

1 was dissolved in dry dichloromethane. Then 2.0 ml TFA were added and the mixture was stirred overnight. 2.0 ml TFA were added and the mixture was stirred one more night. Water was added and the mixture was neutralized with saturated sodium hydrogen carbonate solution. The aqueous layer was extracted twice with dichloromethane and twice with ethyl acetate. The combined organic layers were dried over sodium sulfate and evaporated in vacuum. Further purification was achieved by preparative HPLC: Phenomenex Luna Phenyl-Hexyl: MeCN/H₂O/TFA 40:60:0.1. The aqueous layer was carefully basified to pH 6 with saturated sodium hydrogen carbonate solution and acetonitrile was removed in vacuum. Then brine was added and the aqueous layer was extracted several times with ethylacetate. The combined organic layers were dried over sodium sulfate and evaporated in vacuum. The oily residue was treated with diethyl ether and dried in vacuum and a white foam was obtained.
**Yield:**
0.5 g (M 480.6), 1.05 mmol
m.p.; 48-50°C. MS (ESI) m/z 481.2227 (M+H)⁺.
¹H-NMR (DMSO-d6): Figure 4

### Reference Example 5. O-Cyclopropanoyl-Mirabegron

Step a corresponds to step a of Example 4.

### Step b: O-Cyclopropanoyl-N,N',N'-Mirabegron-tris(t-butylcarbamate)

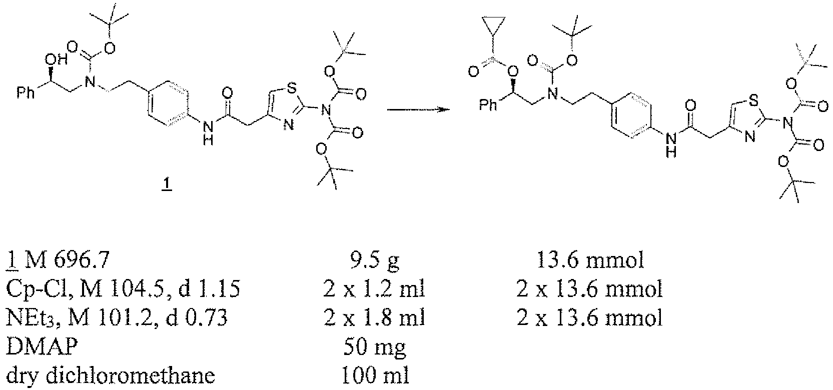

1 was dissolved in dry dichloromethane. Then triethylamine, cyclopropanoyl chloride (Cp-Cl) and DMAP were added and the reaction was stirred overnight. The reaction mixture was quenched with 100 ml water and brine was added. The aqueous layer was extracted twice with dichloromethane. The combined organic layers were evaporated in vacuum. Purification was achieved by repeated preparative HPLC: Phenomenex Luna Phenyl-Hexyl: MeCN/H₂O/TFA 80:20:0.1. The aqueous layer was carefully basified to pH 6 with saturated sodium hydrogen carbonate solution and acetonitrile was removed in vacuum. Then brine was added and the aqueous layer was extracted several times with ethylacetate. The combined organic layers were dried over sodium sulfate and evaporated in vacuum.
**Yield:**
3.5 g (M 764.9), 4.6 mmol

### Step c: O-Cyclopropanoyl-Mirabegron

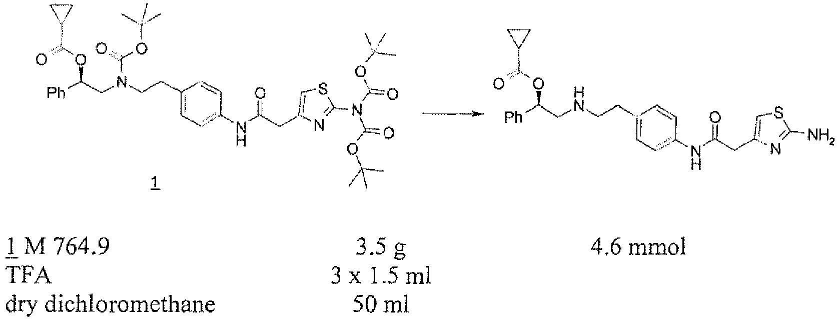

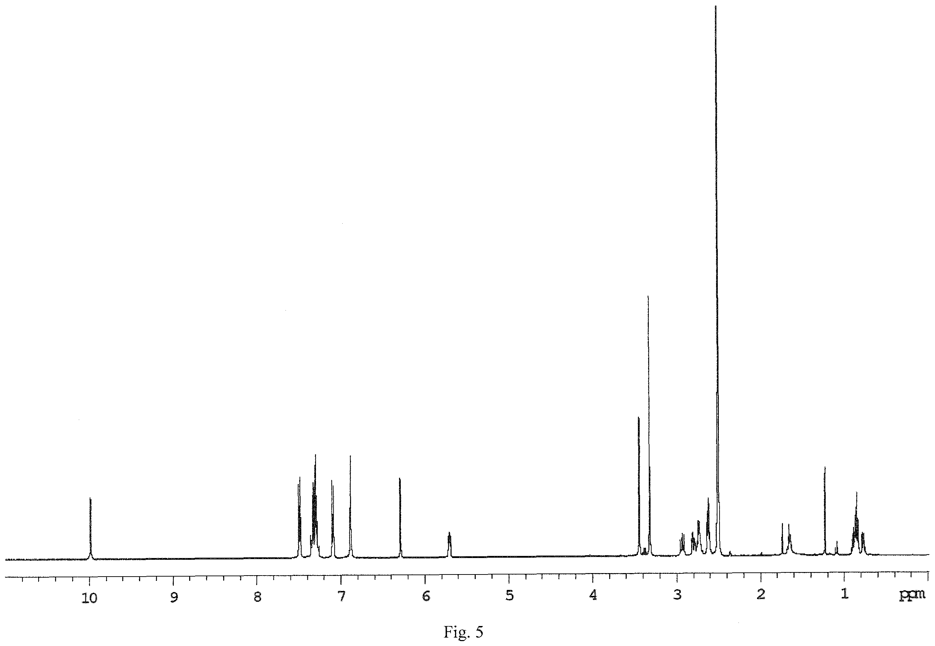

1 was dissolved in dry dichloromethane. Then 1.5 ml TFA were added and the mixture was stirred overnight. Then 1.5 ml TFA were added and once more 1.5 ml TFA and the mixture was stirred one more night. Water was added and the mixture was neutralized with saturated sodium hydrogen carbonate solution. The aqueous layer was extracted twice with dichloromethane and twice with ethyl acetate, The combined organic layers were dried over sodium sulfate and evaporated in vacuum, Further purification was achieved by repeated preparative HPLC: Phenomenex Luna Phenyl-Hexyl: MeCN/H₂O/TFA 30:70:0.1. The aqueous layer was carefully basified to pH 6 with saturated sodium hydrogen carbonate solution and acetonitrile was removed in vacuum. Then brine was added and the aqueous layer was extracted several times with ethylacetate. The combined organic layers were dried over sodium sulfate and evaporated in vacuum. The residue was treated with diethyl ether and dried in vacuum and a white solid was obtained.
**Yield:**
0.2 g (M 464.6), 0.43 mmol
m.p.: 44-47°C. MS (ESI) m/z 465.1917 (M+H)⁺.
¹H-NMR (DMSO-d6): Figure 5

### Reference Example 6. O-Benzoyl-Mirabegron

### Step a: N,N',N'-Mirabegron-tris(t-butylcarbamate)

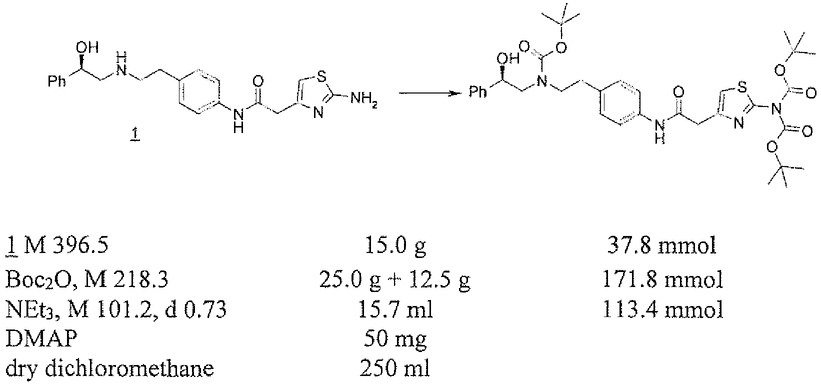

Mirabegron was dissolved in dry dichloromethane. Then triethylamine and Boc₂O were added. DMAP was added and the reaction was stirred overnight. Additional Boc₂O was added and the mixture was stirred one more night. The reaction mixture was quenched with 500 ml water and brine was added. The aqueous layer was extracted twice with dichloromethane. The combined organic layers were evaporated in vacuum. Purification was achieved by repeated column chromatography: silica gel: toluene/ethylacetate 1:1.
**Yield:**
13.7 g (M 696.9), 19.7 mmol

### Step b: O-Benzoyl-N,N',N'-Mirabegron-tris(t-butylcarbamate)

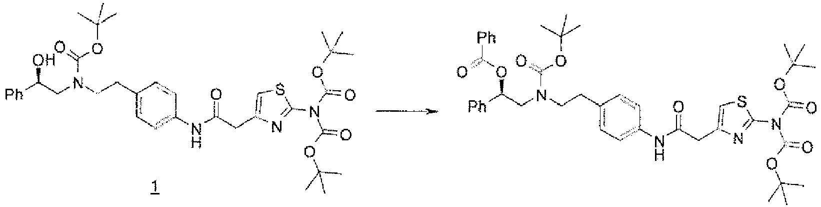

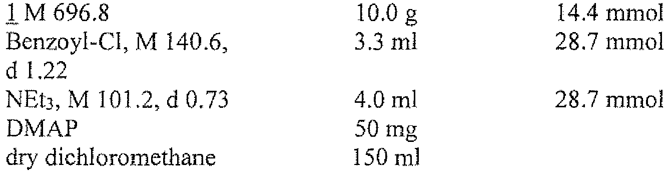

1 was dissolved in dry dichloromethane. Then triethylamine, Benzoyl-Cl and DMAP were added and the reaction was stirred overnight. The reaction mixture was quenched with 100 ml water and brine was added. The aqueous layer was extracted twice with dichloromethane. The combined organic layers were evaporated in vacuum. Purification was achieved by preparative HPLC: Phenomenex Luna Phenyl-Hexyl: MeCN/H₂O/TFA 85:15:0.1. Acetonitrile was removed in vacuum and the aqueous layer was carefully basified to pH 6 with saturated sodium hydrogen carbonate solution. Then brine was added and the aqueous layer was extracted several times with ethylacetate. The combined organic layers were dried over sodium sulfate and evaporated in vacuum.
**Yield:**
8.0 g (M 801.0), 10.0 mmol

### Step c: O-Benzoyl-Mirabegron

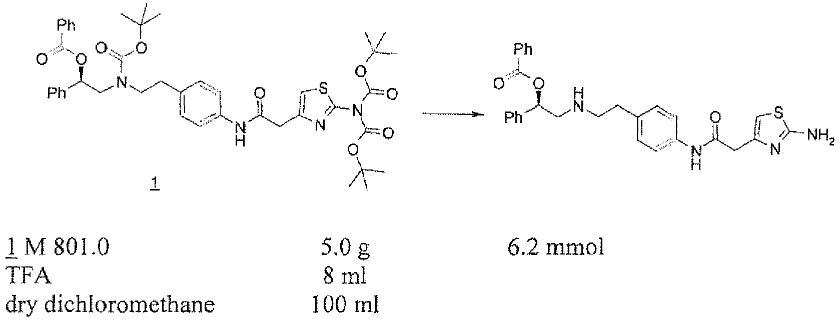

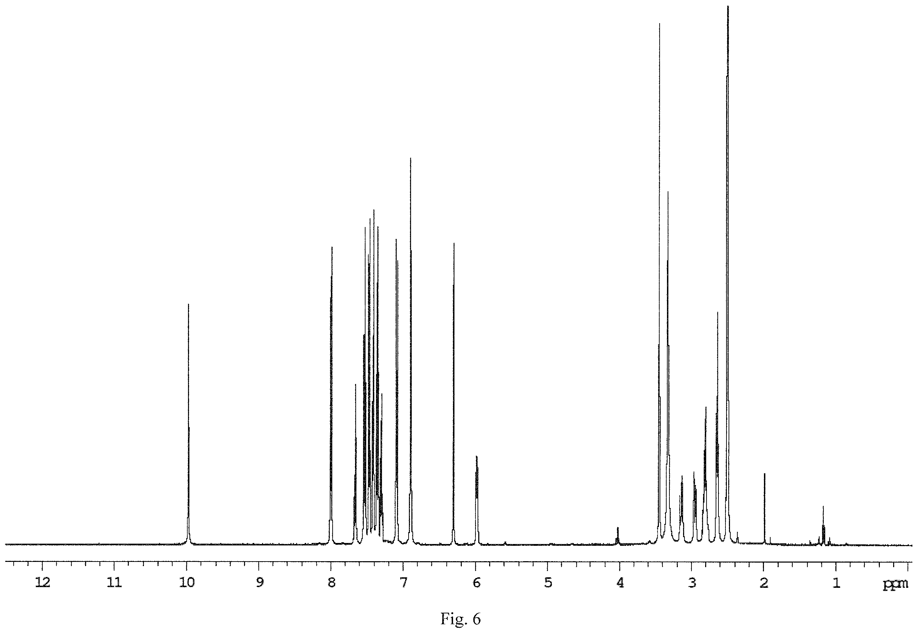

1 was dissolved in dry dichloromethane. Then 4.0 ml TFA were added and the mixture was stirred overnight. 2.0 ml TFA were added and the mixture was stirred one more night. 2.0 ml TFA were added and the mixture was stirred one more night. The solvent and TFA were removed in vacuum. Further purification was achieved by preparative HPLC: Phenomenex Luna Phenyl-Hexyl: MeCN/H₂O/TFA 30:70:0.1. Acetonitrile was removed in vacuum. Then brine and sodium hydrogen carbonate solution was added and the aqueous layer was extracted several times with ethylacetate. The combined organic layers were dried over sodium sulfate and evaporated in vacuum. The residue was treated with diethyl ether and dried in vacuum and a white foam was obtained.
**Yield:**
1.1 g (M 500.6), 2.2 mmol
m.p.: 59-64°C. MS (ESI) m/z 501.1962 (M+H)⁺.
¹H-NMR (DMSO-d6): Figure 6

### Reference Example 7. O-Propanoyl-Mirabegron trifluoroacetate

Step a corresponds to step a of Example 6.

### Step b: O-Propanoyl-N,N',N-Mirabegron-tris(t-butylcarbamate)

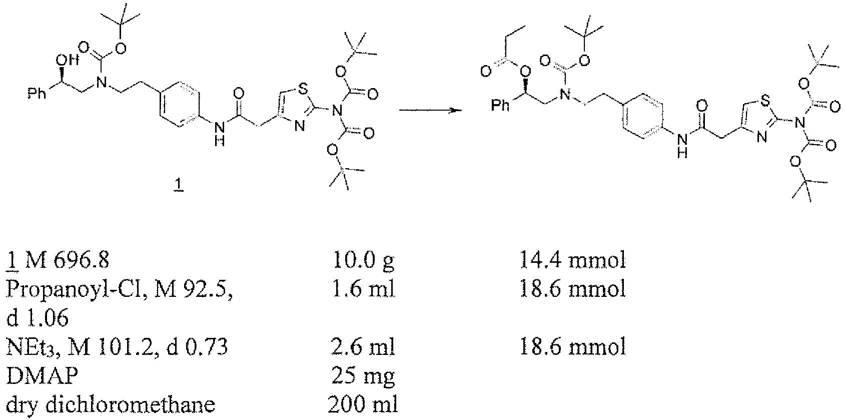

1 was dissolved in dry dichloromethane. Then triethylamine, Propanoyl-Cl and DMAP were added and the reaction was stirred overnight. The reaction mixture was quenched with 100 ml water and brine was added. The aqueous layer was extracted twice with dichloromethane. The combined organic layers were evaporated in vacuum. Purification was achieved by preparative HPLC: Phenomenex Luna Phenyl-Hexyl: MeCN/H₂O/TFA 85:15:0.1. Acetonitrile was removed in vacuum and the aqueous layer was carefully basified to pH 6 with saturated sodium hydrogen carbonate solution. Then brine was added and the aqueous layer was extracted several times with ethylacetate. The combined organic layers were dried over sodium sulfate and evaporated in vacuum.
**Yield:**
4.2 g (M 752.9), 5.6 mmol

### Step c: O-Propanoyl-Mirabegron trifluoroacetate

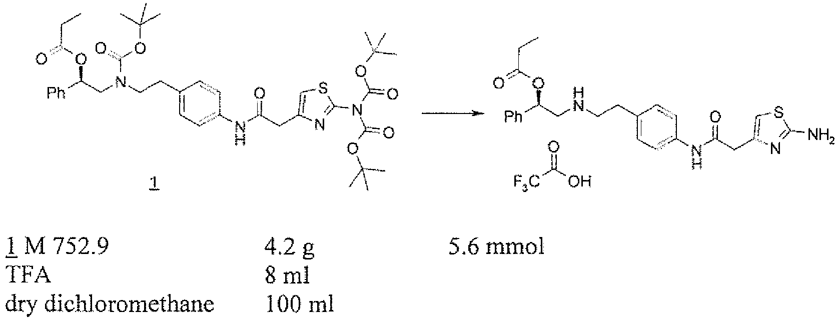

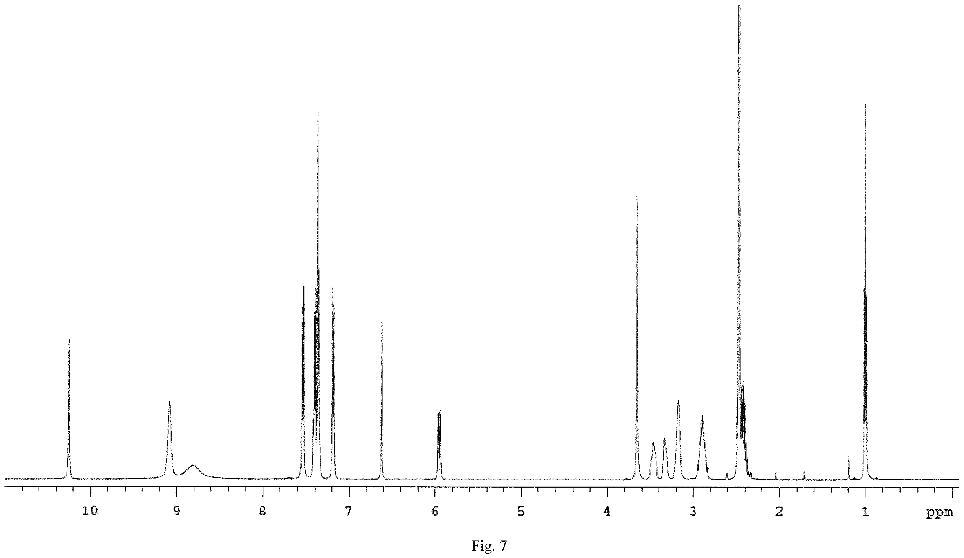

1 was dissolved in dry dichloromethane. Then 4.0 ml TFA were added and the mixture was stirred overnight. 2.0 ml TFA were added and the mixture was stirred one more night. 2.0 ml TFA were added and the mixture was stirred one more night. The solvent and TFA were removed in vacuum. Further purification was achieved by preparative HPLC: Phenomenex Luna Phenyl-Hexyl: MeCN/H₂O/TFA 30:70:0.1 Acetonitrile was removed in vacuum. Then brine and sodium hydrogen carbonate solution was added and the aqueous layer was extracted several times with ethylacetate. The combined organic layers were dried over sodium sulfate and evaporated in vacuum. The residue was treated with diethyl ether and dried in vacuum (decomposition; purity by HPLC is 80%). Purification was achieved once more by preparative HPLC: Phenomenex Luna Phenyl-Hexyl: MeCN/H₂O/TFA 30:70:0.1. Acetonitrile was removed in vacuum. The aqueous residue was lyophilized and white crystals were obtained.
**Yield:**
1.0 g (M 566.6)
m.p.: 70-75°C. MS (ESI) m/z 453.1957 (M-CF₃COOH+H)⁺.
¹H-NMR (DMSO-d6): Figure 7

## Claims

1. A prodrug of mirabegron represented by the following formula (I) 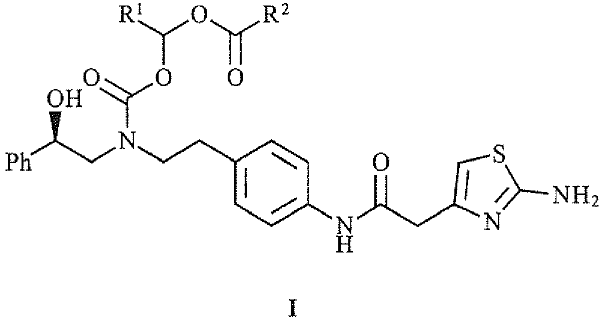 wherein
- R¹ represents hydrogen or an optionally substituted, saturated or unsaturated alkyl, and
- R² represents an optionally substituted, saturated or unsaturated alkyl, optionally substituted, saturated or unsaturated cycloalkyl, optionally substituted, saturated or unsaturated (cycloalkyl)alkyl, optionally substituted, saturated or unsaturated heterocyclyl, optionally substituted, saturated or unsaturated (heterocyclyl)alkyl, optionally substituted aryl, or optionally substituted heteroaryl,
wherein the substituent of the optionally substituted alkyl, cycloalkyl, (cycloalkyl)alkyl, heterocyclyl, (heterocyclyl)alkyl, aryl and heteroaryl is selected from halogen (F, Cl, Br or I), C₁₋₆-alkoxy, preferably C₁₋₄-alkoxy, more preferably C₁₋₂-alkoxy, phenoxy, (C₁₋₄ alkyl)₂ amino and phenyl,
or a pharmaceutically acceptable salt thereof or a solvate thereof.

2. Prodrug according to claim 1, wherein
the saturated alkyl group is C₁₋₇ alkyl, preferably C₁₋₅ alkyl, the unsaturated alkyl group is C₂₋₇ alkenyl, preferably C₂₋₅ alkenyl, the saturated or unsaturated cycloalkyl group is cyclo-C₃₋₆ alkyl, the saturated or unsaturated (cycloalkyl)alkyl group is (cyclo-C₃₋₆ alkyl)C₁₋₅ alkyl, the saturated or unsaturated heterocyclyl group is cyclo-C₃₋₆ heterocyclyl, in which 1 to 3 carbon atoms of the ring are replaced by O, N or S, the saturated or unsaturated (heterocyclyl)alkyl group is (cyclo-C₃₋₆ heterocyclyl)C₁₋₅ alkyl, in which 1 to 3 carbon atoms of the ring are replaced by O, N or S, the aryl group is C₆ or C₁₀ aryl, and the heteroaryl group is C₆ or C₁₀ heteroaryl, in which 1 to 3 carbon atoms of the ring are replaced by O, N or S.

3. Prodrug according to claim 1 or 2, wherein
- R¹ represents hydrogen or an unsubstituted, saturated alkyl, and
- R² represents an unsubstituted, saturated alkyl, or an unsubstituted aryl.

4. Prodrug according to claim 3, wherein
- R¹ is selected from hydrogen, methyl, ethyl, n-propyl, iso-propyl, n-butyl, sec-butyl and tert-butyl, and
- R² is selected from methyl, ethyl, propyl (n- or iso-), butyl (n-, sec- or tert-), n-pentyl, 2-methylbutyl, 3-methylbutyl and phenyl.

5. Prodrug according to claim 4, wherein
- R¹ is hydrogen or methyl, and
- R² is selected from methyl, ethyl, n-propyl, iso-propyl, n-butyl, sec-butyl, n-pentyl and phenyl.

6. Prodrug according to claim 5, wherein
- R¹ is methyl, and
- R² is iso-propyl.

7. Prodrug according to claim 5, wherein
- R¹ is hydrogen, and
- R² is n-pentyl or phenyl.

8. A process for preparing a prodrug according to any one of claims 1-7, wherein the process comprises the step of reacting mirabegron and a carbonate compound represented by the following formula (5) 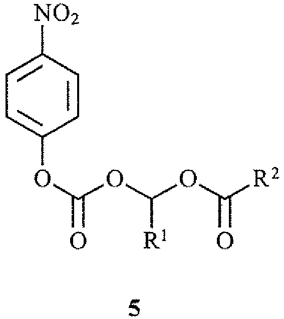 wherein R¹ and R² are as defined above.

9. The process according to claim 8, wherein the carbonate compound 5 is prepared by reacting a carbonate compound and an acid of the following formulas 3 and 4 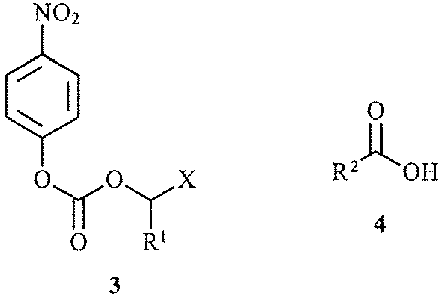 wherein R¹ and R² are as defined above, and X is a leaving group, e.g. Cl, Br, I, alkylsulfonyloxy or arylsulfonyloxy.

10. The process according to claim 9, wherein the carbonate compound 3 is prepared by reacting p-nitrophenol and a compound of the following formula 2 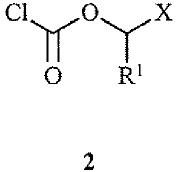 wherein R¹ and X are as defined above.

11. A pharmaceutical composition for oral administration comprising a prodrug according to any one of claims 1-7.

12. A pharmaceutical composition according to claim 11 for use in a method of treating overactive bladder.

13. A prodrug according to any one of claims 1-7 for use in a method of treating overactive bladder.

## Patentansprüche

1. Ein Prodrug von Mirabegron der nachstehenden Formel (I) 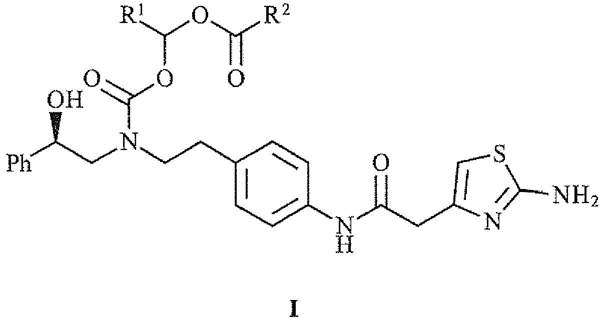 wobei
- R¹ Wasserstoff oder eine gegebenenfalls substituierte, gesättigte oder ungesättigte Alkyl-Gruppe darstellt, und
- R² eine gegebenenfalls substituierte, gesättigte oder ungesättigte Alkyl-Gruppe, gegebenenfalls substituierte, gesättigte oder ungesättigte Cycloalkyl-Gruppe, gegebenenfalls substituierte, gesättigte oder ungesättigte (Cycloalkyl)alkyl-Gruppe, gegebenenfalls substituierte, gesättigte oder ungesättigte Heterocyclyl-Gruppe, gegebenenfalls substituierte, gesättigte oder ungesättigte (Heterocyclyl)alkyl-Gruppe, gegebenenfalls substituierte Aryl-Gruppe oder gegebenenfalls substituierte Heteroaryl-Gruppe darstellt,
wobei die gegebenenfalls substituierte Alkyl-, Cycloalkyl-, (Cycloalkyl)alkyl-, Heterocyclyl-, (Heterocyclyl)alkyl-, Aryl- und Heteroaryl-Gruppe ausgewählt ist aus Halogen (F, Cl, Br oder I), C₁₋₆-Alkoxy, vorzugsweise C₁₋₄-Alkoxy, mehr bevorzugt C₁₋₂-Alkoxy, Phenoxy, (C₁₋₄-Alkyl)₂amino und Phenyl,
oder ein pharmazeutisch verträgliches Salz oder Solvat davon.

2. Prodrug gemäß Anspruch 1, wobei
die gesättigte Alkyl-Gruppe C₁₋₇-Alkyl, vorzugsweise C₁₋₅-Alkyl, die ungesättigte Alkyl-Gruppe C₂₋₇-Alkenyl, vorzugsweise C₂₋₅-Alkenyl, die gesättigte oder ungesättigte Cycloalkyl-Gruppe Cyclo-C₃₋₆-alkyl, die gesättigte oder ungesättigte (Cycloalkyl)alkyl-Gruppe (Cyclo-C₃₋₆-alkyl)C₁₋₅-alkyl, die gesättigte oder ungesättigte Heterocyclyl-Gruppe Cyclo-C₃₋₆-heterocyclyl, wobei 1 bis 3 Kohlenstoffatome des Rings durch O, N oder S substituiert sein können, die gesättigte oder ungesättigte (Heterocyclyl)alkyl-Gruppe (Cyclo-C₃₋₆-heterocyclyl)C₁₋₅-alkyl, wobei 1 bis 3 Kohlenstoffatome des Rings durch O, N oder S substituiert sein können, die Aryl-Gruppe C₆₋ oder C₁₀-Aryl und die Heteroaryl-Gruppe C₆- oder C₁₀-Heteroaryl, wobei 1 bis 3 Kohlenstoffatome des Rings durch O, N oder S substituiert sein können, ist.

3. Prodrug gemäß Anspruch 1 oder 2, wobei
- R¹ Wasserstoff oder eine unsubstituierte, gesättigte Alkyl-Gruppe darstellt und
- R² eine unsubstituierte, gesättigte Alkyl-Gruppe oder eine unsubstituierte Aryl-Gruppe dasrstellt.

4. Prodrug gemäß Anspruch 3, wobei
- R¹ ausgewählt ist aus Wasserstoff, Methyl, Ethyl, n-Propyl, iso-Propyl, n-Butyl, sec-Butyl und tert-Butyl und
- R² ausgewählt ist aus Methyl, Ethyl, Propyl (n- oder iso-), Butyl (n-, sec- oder tert-), n-Pentyl, 2-Methylbutyl, 3-Methylbutyl und Phenyl.

5. Prodrug gemäß Anspruch 4, wobei
- R¹ Wasserstoff oder Methyl ist und
- R² ausgewählt ist aus Methyl, Ethyl, n-Propyl, iso-Propyl, n-Butyl, sec-Butyl, n-Pentyl und Phenyl.

6. Prodrug gemäß Anspruch 5, wobei
- R¹ Methyl ist und
- R² iso-Propyl ist.

7. Prodrug gemäß Anspruch 5, wobei
- R¹ Wasserstoff ist und
- R² n-Pentyl oder Phenyl ist.

8. Verfahren zur Herstellung eines Prodrugs gemäß einem der Ansprüche 1-7, wobei das Verfahren den Schritt umfasst: Reaktion von Mirabegron und einer Carbonat-Verbindung der nachstehenden Formel (5) 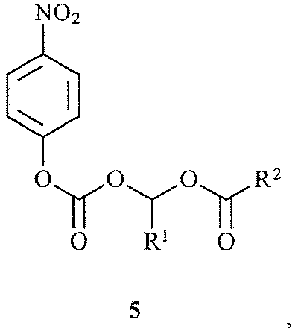 wobei R¹ und R² wie vorstehend definiert sind.

9. Verfahren gemäß Anspruch 8, wobei die Carbonat-Verbindung 5 durch Reaktion einer Carbonat-Verbindung und einer Säure der nachstehenden Formeln 3 und 4 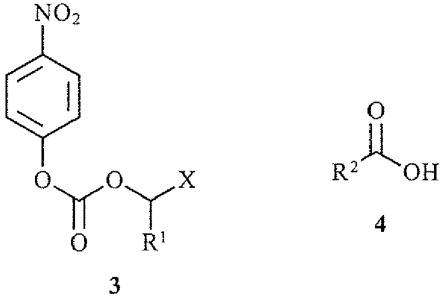 hergestellt wird, wobei R¹ und R² wie vorstehend definiert sind und X eine Abgangsgruppe ist, z.B. Cl, Br, I, Alkylsulfonyloxy oder Arylsulfonyloxy.

10. Verfahren gemäß Anspruch 9, wobei die Carbonat-Verbindung 3 durch Reaktion von p-Nitrophenol mit einer Verbindung der nachstehenden Formel 2 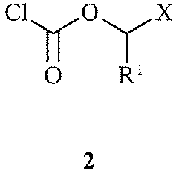 wobei R¹ und X wie vorstehend definiert sind, hergestellt wird.

11. Pharmazeutische Zusammensetzung zur oralen Verabreichung, enthaltend ein Prodrug gemäß einem der Ansprüche 1-7.

12. Pharmazeutische Zusammensetzung gemäß Anspruch 11 zur Verwendung in einem Verfahren zur Behandlung der überaktiven Blase.

13. Prodrug gemäß einem der Ansprüche 1-7 zur Verwendung in einem Verfahren zur Behandlung der überaktiven Blase.

## Revendications

1. Promédicament de mirabegron représenté par la formule suivante (I) 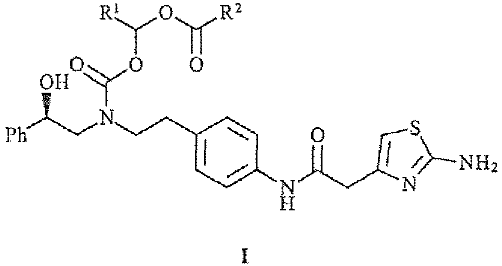 dans lequel
- R¹ représente un hydrogène ou un alkyle saturé ou insaturé facultativement substitué, et
- R² représente un alkyle saturé ou insaturé facultativement substitué, un cycloalkyle saturé ou insaturé facultativement substitué, un (cycloalkyl)alkyle saturé ou insaturé facultativement substitué, un hétérocyclyle saturé ou insaturé facultativement substitué, un (hétérocyclyl)alkyle saturé ou insaturé facultativement substitué, un aryle facultativement substitué, ou un hétéroaryle facultativement substitué,
dans lequel le substituant de l'alkyle, du cycloalkyle, du (cycloalkyl)alkyle, de l'hétérocyclyle, de l'(hétérocyclyl)alkyle, de l'aryle et de l'hétéroaryle facultativement substitué est choisi parmi un halogène (F, Cl, Br ou I), un alcoxy en C₁₋₆, de préférence un alcoxy en C₁₋₄, de manière plus préférée un alcoxy en C₁₋₂, un phénoxy, un amino(alkyle en C₁₋₄) et un phényle, ou un sel pharmaceutiquement acceptable de ceux-ci ou un solvate de ceux-ci.

2. Promédicament selon la revendication 1, dans lequel
le groupe alkyle saturé est un alkyle en C₁₋₇, de préférence un alkyle en C₁₋₅, le groupe alkyle insaturé est un alcényle en C₂₋₇, de préférence un alcényle en C₂₋₅, le groupe cycloalkyle saturé ou insaturé est un cycloalkyle en C₃₋₆, le groupe (cycloalkyl)alkyle saturé ou insaturé est un (cycloalkyle en C₃₋₆₎)alkyle en C₁₋₅, le groupe hétérocyclyle saturé ou insaturé est un groupe cyclo-hétérocyclyle en C₃₋₆, dans lequel 1 à 3 atomes de carbone du cycle sont remplacés par O, N ou S, le groupe (hétérocyclyl)alkyle saturé ou insaturé est un (cyclo-hétérocyclyle en C₃₋₆) alkyle en C₁₋₅, dans lequel 1 à 3 atomes de carbone du cycle sont remplacés par O, N ou S, le groupe aryle est un groupe aryle en C₆ ou C₁₀ et le groupe hétéroaryle est un groupe hétéroaryle en C₆ ou C₁₀, dans lequel 1 à 3 atomes de carbone du cycle sont remplacés par O, N ou S.

3. Promédicament selon la revendication 1 ou 2, dans lequel
- R1 représente un hydrogène ou un groupe alkyle saturé non substitué, et
- R2 représente un groupe alkyle saturé non substitué ou un groupe aryle non substitué.

4. Promédicament selon la revendication 3, dans lequel
- R¹ est choisi parmi hydrogène, méthyle, éthyle, n-propyle, isopropyle, n-butyle, sec-butyle et tert-butyle, et
- R² est choisi parmi méthyle, éthyle, (n- ou iso-)propyle, (n-, sec- ou tert-)butyle, n-pentyle, 2-méthylbutyle, 3-méthylbutyle et phényle.

5. Promédicament selon la revendication 4, dans lequel
- R¹ est un hydrogène ou un méthyle, et
- R² est choisi parmi méthyle, éthyle, n-propyle, isopropyle, n-butyle, sec-butyle, n-pentyle et phényle.

6. Promédicament selon la revendication 5, dans lequel
- R¹ est un méthyle, et
- R² est un isopropyle.

7. Promédicament selon la revendication 5, dans lequel
- R¹ est un hydrogène, et
- R² est un n-pentyle ou phényle.

8. Procédé de préparation d'un promédicament selon l'une quelconque des revendications 1 à 7, dans lequel le procédé comprend l'étape consistant à faire réagir le mirabegron et un composé de carbonate représenté par la formule (5) suivante 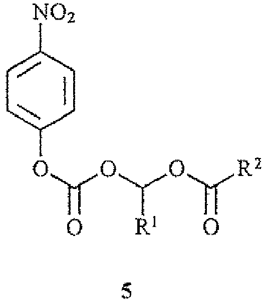 dans lequel R¹ et R² sont tels que définis ci-dessus.

9. Procédé selon la revendication 8, dans lequel le composé de carbonate 5 est préparé en faisant réagir un composé de carbonate et un acide de formules 3 et 4 suivantes 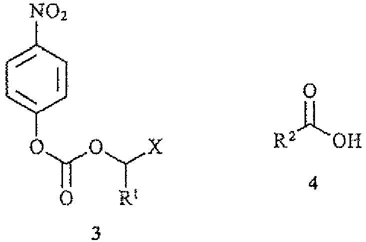 dans lequel R¹ et R² sont tels que définis ci-dessus, et X est un groupe partant, par exemple Cl, Br, I, alkylsulfonyloxy ou arylsulfonyloxy.

10. Procédé selon la revendication 9, dans lequel le composé de carbonate 3 est préparé en faisant réagir du p-nitrophénol et un composé de la formule 2 suivante 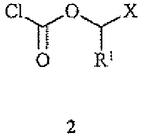 dans lequel R¹ et X sont tels que définis ci-dessus.

11. Composition pharmaceutique pour administration orale comprenant un promédicament selon l'une quelconque des revendications 1 à 7.

12. Composition pharmaceutique selon la revendication 11, destinée à être utilisée dans un procédé de traitement d'une vessie hyperactive.

13. Promédicament selon l'une quelconque des revendications 1 à 7, destiné à être utilisé dans un procédé de traitement d'une vessie hyperactive.
